# EUROPEAN PATENT APPLICATION

(11) **EP 1 174 502 A1**
(43) Date of publication of application: **23.01.2002**
(21) Application number: 00306087.8
(22) Date of filing: 18.07.2000
(51) Int. Cl.: C12N 15/12, C12N 15/63, C07K 14/47, C07K 16/18, C12Q 1/68, G01N 33/53

(54) **Type 2 dendritic cell precursor derived coding nucleic acids and related compositions and methods**

(71) Applicant: SCHERING CORPORATION, Kenilworth, New Jersey 07033-0530 (US)
(72) Inventor: Rissoan, Marie-Clotilde, 69005 Lyon (FR); Bridon, Jean-Michel, 69340 Francheville (FR); Duhen, Thomas, 69005 Lyon (FR); Briere, Francine, 69210 Germain Sur 'arbresie (FR); Bates, Elizabeth, 69001 Lyon (FR)
(74) Representative: Ritter, Stephen David

(57) **Abstract**

Genes related to dendritic cells of the immune system have been isolated, cloned, sequenced, and functionally identified. These nucleic acids and encoded proteins can be used for diagnostic and therapeutic purposes.

SEQUENCE LISTING

<110> Bates, Elizabeth
      Bridon, Jean-Michel
      Briere, Francine
      Duhen, Thomas
      Rissoan, Marie-Clothilde

<120> Dendritic Cell-Derived Nucleic Acids and Related
      Compositions and Methods

<130> SF01190

<140>
<141>

<160> 12

<170> PatentIn Ver. 2.1

<210> 1
<211> 728
<212> DNA
<213> Homo sapiens

<220>
<221> CDS
<222> (104)..(448)

<400> 1
acgtagtttt catttgggtg agattctctc ccaggccaca agacatttcc tgctcggaac 60

cttgtttact aatttccact gcttttaagg ccctgcactg aaa atg caa gct cag   115
                                                Met Gln Ala Gln
                                                  1

gcg ccg gtg gtc gtt gtg acc caa cct gga gtc ggt ccc ggt ccg gcc   163
Ala Pro Val Val Val Val Thr Gln Pro Gly Val Gly Pro Gly Pro Ala
  5                  10                  15                  20

ccc cag aac tcc aac tgg cag aca ggc atg tgt gac tgt ttc agc gac   211
Pro Gln Asn Ser Asn Trp Gln Thr Gly Met Cys Asp Cys Phe Ser Asp
                 25                  30                  35

tgc gga gtc tgt ctc tgt ggc aca ttt tgt ttc ccg tgc ctt ggg tgt   259
Cys Gly Val Cys Leu Cys Gly Thr Phe Cys Phe Pro Cys Leu Gly Cys
             40                  45                  50

caa gtt gca gct gat atg aat gaa tgc tgt ctg tgt gga aca agc gtc   307
Gln Val Ala Ala Asp Met Asn Glu Cys Cys Leu Cys Gly Thr Ser Val
         55                  60                  65

gca atg agg act ctc tac agg acc cga tat ggc atc cct gga tct att   355
Ala Met Arg Thr Leu Tyr Arg Thr Arg Tyr Gly Ile Pro Gly Ser Ile
     70                  75                  80

tgt gat gac tat atg gca act ctt tgc tgt cct cat tgt act ctt tgc   403
Cys Asp Asp Tyr Met Ala Thr Leu Cys Cys Pro His Cys Thr Leu Cys
 85                  90                  95                 100

caa atc aag aga gat atc aac aga agg aga gcc atg cgt act ttc       448

Gln Ile Lys Arg Asp Ile Asn Arg Arg Arg Ala Met Arg Thr Phe
                105                 110                 115

taaaaactga tggtgaaaag ctcttaccga agcaacaaaa ttcagcagac acctcttcag 508

cttgagttct tcaccatctt ttgcaactga aatatgatgg atatgcttaa gtacaactga 568

tggcatgaaa aaaatcaaat ttttgattta ttataaatga atgttgtccc tgaacttagc 628

taaatggtgc aacttagttt ctccttgctt tcatattatc gaatttcctg gcttataaac 688

tttttaaatt acatttgaaa tataaaccaa atgaaatatt                       728

<210> 2
<211> 115
<212> PRT
<213> Homo sapiens

<400> 2
Met Gln Ala Gln Ala Pro Val Val Val Val Thr Gln Pro Gly Val Gly
  1               5                  10                  15

Pro Gly Pro Ala Pro Gln Asn Ser Asn Trp Gln Thr Gly Met Cys Asp
             20                  25                  30

Cys Phe Ser Asp Cys Gly Val Cys Leu Cys Gly Thr Phe Cys Phe Pro
         35                  40                  45

Cys Leu Gly Cys Gln Val Ala Ala Asp Met Asn Glu Cys Cys Leu Cys
     50                  55                  60

Gly Thr Ser Val Ala Met Arg Thr Leu Tyr Arg Thr Arg Tyr Gly Ile
 65                  70                  75                  80

Pro Gly Ser Ile Cys Asp Asp Tyr Met Ala Thr Leu Cys Cys Pro His
                 85                  90                  95

Cys Thr Leu Cys Gln Ile Lys Arg Asp Ile Asn Arg Arg Arg Ala Met
            100                 105                 110

Arg Thr Phe
        115

<210> 3
<211> 19
<212> DNA
<213> Artificial Sequence

<220>
<223> Description of Artificial Sequence:  Primer

<400> 3
ccaaagagta caatgagga                                               19

<210> 4
<211> 19
<212> DNA

<213> Artificial Sequence

<220>
<223> Description of Artificial Sequence: Primer

<400> 4
gccacaagac atttcctgc                                                    19


<210> 5
<211> 2101
<212> DNA
<213> Homo sapiens

<220>
<221> CDS
<222> (330)..(1244)

<400> 5
aggcaaccgc ttatttgcat agggtcccgt cctggccaac gagggcgccc caaatgttca 60

ggacatagaa gaaggggtta actggcccgg atctcctcct cgccttccaa gcccgctaag 120

cactggggtt atctacccat tccccagaag gggagactga ggcagcccac cagccaaagg 180

aggcgaccag actggggctg cgttttacca tttcagaagc ggcttgagct ggtctgagct 240

ataataataa acactggcgg tggaggcgag ggcgaccaca gggctgaggt cagggctagg 300

attccggtgt ctctacgtag gttgcttga atg ggg ggc acc ctg gca tgg acg   353
                                Met Gly Gly Thr Leu Ala Trp Thr
                                1               5

ctg ctg ttg ccg ctg ctg ctg cgg gag tca gac agc cta gaa ccg tcg   401
Leu Leu Leu Pro Leu Leu Leu Arg Glu Ser Asp Ser Leu Glu Pro Ser
    10                  15                  20

tgc acc gtg tcc tcc gcg gat gtg gac tgg aac gcg gag ttc agt gcc   449
Cys Thr Val Ser Ser Ala Asp Val Asp Trp Asn Ala Glu Phe Ser Ala
25                  30                  35                  40

acg tgc ctg aaw ttc agt ggc ctc agc ctg agc ctg cct cac aac cag   497
Thr Cys Leu Xaa Phe Ser Gly Leu Ser Leu Ser Leu Pro His Asn Gln
                45                  50                  55

tct ctg cgg gcc agc aac gtg awt ctc ctt gac ctg tct ggg aac ggc   545
Ser Leu Arg Ala Ser Asn Val Xaa Leu Leu Asp Leu Ser Gly Asn Gly
            60                  65                  70

ctg cga gag ctt cca gtg acc ttc ttt ggc cac ctg cag aag ctt gag   593
Leu Arg Glu Leu Pro Val Thr Phe Phe Gly His Leu Gln Lys Leu Glu
        75                  80                  85

gtc ctg aac gtg cta cgc aac ccc ttg tct cgt gtg gat ggg gcg ctg   641
Val Leu Asn Val Leu Arg Asn Pro Leu Ser Arg Val Asp Gly Ala Leu
    90                  95                  100

gcc gcc cgc tgt gac ctt gac ctg cag gcc gac tgc aac tgt gcc ctg   689
Ala Ala Arg Cys Asp Leu Asp Leu Gln Ala Asp Cys Asn Cys Ala Leu
105                 110                 115                 120

gag tcc tgg cac gac atc cgc cga gac aac tgc tct ggc cag aag cct 737
Glu Ser Trp His Asp Ile Arg Arg Asp Asn Cys Ser Gly Gln Lys Pro
                125                 130                 135

ctg ctc tgc tgg gac aca acc agc tcc cag cac aac ctc tct gcc ttc 785
Leu Leu Cys Trp Asp Thr Thr Ser Ser Gln His Asn Leu Ser Ala Phe
            140                 145                 150

ctg gag gtc agc tgc gcc cct ggc ctg gcc tct gca act atc ggg gca 833
Leu Glu Val Ser Cys Ala Pro Gly Leu Ala Ser Ala Thr Ile Gly Ala
        155                 160                 165

gtg gtg gtc agc ggg tgc ctg ctt ctt gga ctt gcc atc gct ggc cct 881
Val Val Val Ser Gly Cys Leu Leu Leu Gly Leu Ala Ile Ala Gly Pro
    170                 175                 180

gtg ctg gcc tgg aga ctc tgg cga tgc cga gtg gcc aga agc cgg gag 929
Val Leu Ala Trp Arg Leu Trp Arg Cys Arg Val Ala Arg Ser Arg Glu
185                 190                 195                 200

ctg aac aaa ccc tgg gct gct cag gat ggg ccc aag ccc ggt tta ggc 977
Leu Asn Lys Pro Trp Ala Ala Gln Asp Gly Pro Lys Pro Gly Leu Gly
                205                 210                 215

ttg cag cca cgg tac ggc agc cgg agc gcc ccc aag ccc caa gtg gcc 1025
Leu Gln Pro Arg Tyr Gly Ser Arg Ser Ala Pro Lys Pro Gln Val Ala
            220                 225                 230

gtg cca tcc tgc ccc tcc act ccc gac tat gag aac atg ttt gtg ggc 1073
Val Pro Ser Cys Pro Ser Thr Pro Asp Tyr Glu Asn Met Phe Val Gly
        235                 240                 245

cag cca gca gcc gag cac cag tgg gat gaa caa ggg gct cac cct tca 1121
Gln Pro Ala Ala Glu His Gln Trp Asp Glu Gln Gly Ala His Pro Ser
    250                 255                 260

gag gac aat gac ttt tac atc aac tac aag gac atc gac ctg gct tcc 1169
Glu Asp Asn Asp Phe Tyr Ile Asn Tyr Lys Asp Ile Asp Leu Ala Ser
265                 270                 275                 280

cag cct gtc tac tgt aac ctg cag tca ctg ggc cag gcc tca atg gat 1217
Gln Pro Val Tyr Cys Asn Leu Gln Ser Leu Gly Gln Ala Ser Met Asp
                285                 290                 295

gaa gag gag tac gtg atc ccc ggg cac tgagcctaag atgtcctaac 1264
Glu Glu Glu Tyr Val Ile Pro Gly His
            300                 305

ctccacccag aaccccttca gtccctgctg ggtgactcag ggcgtcctaa ctcctccatg 1324

gcctcagttt ccccatctga agaatgggga caggaaagga ttgtccttga ggccccagga 1384

agctctgccg ccccctccct gtccctcatg ccgctcctca gctccctcag ctcctagagg 1444

gggaagagga gagaccccca acaaggggac aggagggtca ctgtgccaat cctgtcatca 1504

ccctcctgtg gatgtacagg cagtgctcaa taaatgcttc gaggctgatg aggctgctgg 1564

ctcagggtgc gtgggttcct caaggtgggg atttctgagt tctaagacca agtctccatc 1624

tgagactccc aaattgctcc ccacctccca tccctgtttt tttttgttgt tgttgtttgt 1684

ttgtttgttt ttgaaactga gtstcactct gtcacccagg ctggagtgca atgctgcgrt 1744

ctcagctcac tgcaacctcc gccycctggg ttcaagtgat tctgcctgcc tcagcctcct 1804

gagtagctgg gattacagca cccgccacca tgccgagcta atttttgtat ttataataga 1864

gatggggttt cgccatgttg gccaggctgg tctcgaactc ctgacctcaa gcgatctgcc 1924

cgcctcggcc tcctgaagtg ctgggattac aggcgtggcc actgcgccca ggcacattcc 1984

tcccttctgc ccctctcagg gccccttccc aggtccctga tctccaggct tggcctccag 2044

agcagcccac accaacccca aaataaaawa atgtatatat tcmwwwaaaa aaaaaaa 2101

<210> 6
<211> 305
<212> PRT
<213> Homo sapiens

<400> 6
Met Gly Gly Thr Leu Ala Trp Thr Leu Leu Leu Pro Leu Leu Leu Arg
1               5                   10                  15

Glu Ser Asp Ser Leu Glu Pro Ser Cys Thr Val Ser Ser Ala Asp Val
            20                  25                  30

Asp Trp Asn Ala Glu Phe Ser Ala Thr Cys Leu Xaa Phe Ser Gly Leu
        35                  40                  45

Ser Leu Ser Leu Pro His Asn Gln Ser Leu Arg Ala Ser Asn Val Xaa
    50                  55                  60

Leu Leu Asp Leu Ser Gly Asn Gly Leu Arg Glu Leu Pro Val Thr Phe
65                  70                  75                  80

Phe Gly His Leu Gln Lys Leu Glu Val Leu Asn Val Leu Arg Asn Pro
                85                  90                  95

Leu Ser Arg Val Asp Gly Ala Leu Ala Ala Arg Cys Asp Leu Asp Leu
            100                 105                 110

Gln Ala Asp Cys Asn Cys Ala Leu Glu Ser Trp His Asp Ile Arg Arg
        115                 120                 125

Asp Asn Cys Ser Gly Gln Lys Pro Leu Leu Cys Trp Asp Thr Thr Ser
    130                 135                 140

Ser Gln His Asn Leu Ser Ala Phe Leu Glu Val Ser Cys Ala Pro Gly
145                 150                 155                 160

Leu Ala Ser Ala Thr Ile Gly Ala Val Val Val Ser Gly Cys Leu Leu
                165                 170                 175

Leu Gly Leu Ala Ile Ala Gly Pro Val Leu Ala Trp Arg Leu Trp Arg
            180                 185                 190

Cys Arg Val Ala Arg Ser Arg Glu Leu Asn Lys Pro Trp Ala Ala Gln
        195                 200                 205

Asp Gly Pro Lys Pro Gly Leu Gly Leu Gln Pro Arg Tyr Gly Ser Arg
    210                 215                 220

Ser Ala Pro Lys Pro Gln Val Ala Val Pro Ser Cys Pro Ser Thr Pro
225                 230                 235                 240

Asp Tyr Glu Asn Met Phe Val Gly Gln Pro Ala Ala Glu His Gln Trp
                245                 250                 255

Asp Glu Gln Gly Ala His Pro Ser Glu Asp Asn Asp Phe Tyr Ile Asn
            260                 265                 270

Tyr Lys Asp Ile Asp Leu Ala Ser Gln Pro Val Tyr Cys Asn Leu Gln
        275                 280                 285

Ser Leu Gly Gln Ala Ser Met Asp Glu Glu Glu Tyr Val Ile Pro Gly
    290                 295                 300

His
305

<210> 7
<211> 22
<212> DNA
<213> Artificial Sequence

<220>
<223> Description of Artificial Sequence:  Primer

<400> 7
cgagagcttc cagtgacctt ct                                          22

<210> 8
<211> 22
<212> DNA
<213> Artificial Sequence

<220>
<223> Description of Artificial Sequence:  Primer

<400> 8
catgttctca tagtcgggag tg                                          22

<210> 9
<211> 1256
<212> DNA
<213> Homo sapiens

<220>
<221> CDS
<222> (114)..(1010)

<400> 9
ctttccaagg gagtggttgt gtgatcgcca tcttagggaa aagatgttct cgtccgtngc 60

gcacctggcg cgggcgaacc ccttcaacac gccacatctg cagctggtgc acg atg    116
                                                           Met

                                                           1

gtc tcg ggg acc tcc gca gag ctc gga gcg gcg gag gca gag acc gag 164
Val Ser Gly Thr Ser Ala Glu Leu Gly Ala Ala Glu Ala Glu Thr Glu
            5                   10                  15

gct gca ccg gca gag gct gcg ggg cgg acg cgc ggg ccg gcg cac atg 212
Ala Ala Pro Ala Glu Ala Ala Gly Arg Thr Arg Gly Pro Ala His Met
        20                  25                  30

gtg aag att agc ttc cag ccc gcc gtg gct ggc atc aag ggc gac aag 260
Val Lys Ile Ser Phe Gln Pro Ala Val Ala Gly Ile Lys Gly Asp Lys
    35                  40                  45

gct gac aag gcg tcg gcg tcg gcc cct gcg ccg gcc tcg gcc acc gag 308
Ala Asp Lys Ala Ser Ala Ser Ala Pro Ala Pro Ala Ser Ala Thr Glu
50                  55                  60                  65

atc ctg ctg acg ccg gct agg gag gag cag ccc cca caa cat cga tcc 356
Ile Leu Leu Thr Pro Ala Arg Glu Glu Gln Pro Pro Gln His Arg Ser
                70                  75                  80

aag agg ggg agc tca gtg ggc ggc gtg tgc tac ctg tcg atg ggc atg 404
Lys Arg Gly Ser Ser Val Gly Gly Val Cys Tyr Leu Ser Met Gly Met
            85                  90                  95

gtc gtg ctg ctc atg ggc ctc gtg ttc gcc tct gtc tac atc tac aga 452
Val Val Leu Leu Met Gly Leu Val Phe Ala Ser Val Tyr Ile Tyr Arg
        100                 105                 110

tac ttc tty ctt gcr cag ctg gcc cga gat aac ttc ttc cgc tgt ggt 500
Tyr Phe Phe Leu Xaa Gln Leu Ala Arg Asp Asn Phe Phe Arg Cys Gly
    115                 120                 125

gtg ctg tat gag gac tcc ctg tcc tcc cag gtc cgg act cag atg gag 548
Val Leu Tyr Glu Asp Ser Leu Ser Ser Gln Val Arg Thr Gln Met Glu
130                 135                 140                 145

ctg gaa gag gat gtg aaa atc tac ctc gac gag aac tac gag cgc atc 596
Leu Glu Glu Asp Val Lys Ile Tyr Leu Asp Glu Asn Tyr Glu Arg Ile
                150                 155                 160

aac gtg cct gtg ccc cag ttt ggc ggc ggt gac cct gca gac atc atc 644
Asn Val Pro Val Pro Gln Phe Gly Gly Gly Asp Pro Ala Asp Ile Ile
            165                 170                 175

cat gac ttc cag cgg ggt ctg act gcg tac cat gat atc tcc ctg gac 692
His Asp Phe Gln Arg Gly Leu Thr Ala Tyr His Asp Ile Ser Leu Asp
        180                 185                 190

aag tgc tat gtc atc gaa ctc aac acc acc att gtg ctg ccc cct cgc 740
Lys Cys Tyr Val Ile Glu Leu Asn Thr Thr Ile Val Leu Pro Pro Arg
    195                 200                 205

aac ttc tgg gag ctc ctc atg aac gtg aag agg ggg acc tac ctg ccg 788
Asn Phe Trp Glu Leu Leu Met Asn Val Lys Arg Gly Thr Tyr Leu Pro
210                 215                 220                 225

cag acg tac atc atc cag gag gag atg gtg gtc acg gag cat gtc agt 836
Gln Thr Tyr Ile Ile Gln Glu Glu Met Val Val Thr Glu His Val Ser
                230                 235                 240

gac aag gag gcc ctg ggg tcc ttc atc tac cac ctg tgc aac ggg aaa    884
Asp Lys Glu Ala Leu Gly Ser Phe Ile Tyr His Leu Cys Asn Gly Lys
            245                 250                 255

gac acc tac cgg ctc cgg cgc cgg gca acg cgg agg cgr atc aac aag    932
Asp Thr Tyr Arg Leu Arg Arg Arg Ala Thr Arg Arg Xaa Ile Asn Lys
        260                 265                 270

cgt ggg gcc aag aac tgc aat gcc atc cgc cac ttc gag aac acc ttc    980
Arg Gly Ala Lys Asn Cys Asn Ala Ile Arg His Phe Glu Asn Thr Phe
    275                 280                 285

gtg gtg gag acg ctc atc tgc ggg gtg gtg tgaggccctc ctcccccaga     1030
Val Val Glu Thr Leu Ile Cys Gly Val Val
290                 295

accccctgcc gtgttcctct tttcttcttt ccggctgctc tctggccctc ctccttcccc 1090

ctgcttagct tgtactttgg acgcgtttct atcagaggtg acatgtctct ccattcctct 1150

ccaaccctgc ccacctccct gtaccagagc tgtgatctct cggtgggggg cccatytctg 1210

ctgacctggg tgttgcggag gagaggcggt tgtncaaagt gttttt                1256

<210> 10
<211> 299
<212> PRT
<213> Homo sapiens

<400> 10
Met Val Ser Gly Thr Ser Ala Glu Leu Gly Ala Ala Glu Ala Glu Thr
1               5                   10                  15

Glu Ala Ala Pro Ala Glu Ala Ala Gly Arg Thr Arg Gly Pro Ala His
            20                  25                  30

Met Val Lys Ile Ser Phe Gln Pro Ala Val Ala Gly Ile Lys Gly Asp
        35                  40                  45

Lys Ala Asp Lys Ala Ser Ala Ser Ala Pro Ala Pro Ala Ser Ala Thr
    50                  55                  60

Glu Ile Leu Leu Thr Pro Ala Arg Glu Glu Gln Pro Pro Gln His Arg
65                  70                  75                  80

Ser Lys Arg Gly Ser Ser Val Gly Gly Val Cys Tyr Leu Ser Met Gly
                85                  90                  95

Met Val Val Leu Leu Met Gly Leu Val Phe Ala Ser Val Tyr Ile Tyr
            100                 105                 110

Arg Tyr Phe Phe Leu Xaa Gln Leu Ala Arg Asp Asn Phe Phe Arg Cys
        115                 120                 125

Gly Val Leu Tyr Glu Asp Ser Leu Ser Ser Gln Val Arg Thr Gln Met
    130                 135                 140

Glu Leu Glu Glu Asp Val Lys Ile Tyr Leu Asp Glu Asn Tyr Glu Arg
145                 150                 155                 160

Ile Asn Val Pro Val Pro Gln Phe Gly Gly Gly Asp Pro Ala Asp Ile
                165                 170                 175

Ile His Asp Phe Gln Arg Gly Leu Thr Ala Tyr His Asp Ile Ser Leu
            180                 185                 190

Asp Lys Cys Tyr Val Ile Glu Leu Asn Thr Thr Ile Val Leu Pro Pro
        195                 200                 205

Arg Asn Phe Trp Glu Leu Leu Met Asn Val Lys Arg Gly Thr Tyr Leu
    210                 215                 220

Pro Gln Thr Tyr Ile Ile Gln Glu Glu Met Val Val Thr Glu His Val
225                 230                 235                 240

Ser Asp Lys Glu Ala Leu Gly Ser Phe Ile Tyr His Leu Cys Asn Gly
                245                 250                 255

Lys Asp Thr Tyr Arg Leu Arg Arg Arg Ala Thr Arg Arg Xaa Ile Asn
            260                 265                 270

Lys Arg Gly Ala Lys Asn Cys Asn Ala Ile Arg His Phe Glu Asn Thr
        275                 280                 285

Phe Val Val Glu Thr Leu Ile Cys Gly Val Val
    290                 295

<210> 11
<211> 17
<212> DNA
<213> Artificial Sequence

<220>
<223> Description of Artificial Sequence:  Primer

<400> 11
gataacttcc gctgtgg                                                17

<210> 12
<211> 20
<212> DNA
<213> Artificial Sequence

<220>
<223> Description of Artificial Sequence:  Primer

<400> 12
agtcagaccc cgctggaagt                                             20

## Description

### Field of the Invention

The present invention relates generally to genes associated with dendritic cells, cells which function in the immune system. The invention more particularly relates to nucleic acids isolated from dendritic cells, proteins encoded by said nucleic acids, and related diagnostic and therapeutic compositions and methods.

### Background of the Invention

Dendritic cells specialize in the uptake of antigen and the presentation of antigen to T cells. Dendritic cells thus play a critical role in antigen-specific immune responses.

Dendritic cells are represented by a diverse population of morphologically similar cell types distributed widely throughout the body in a variety of lymphoid and non-lymphoid tissues (Caux, *et al.*, 1995, *Immunology Today* **16**:2; Steinman, 1991, *Ann. Rev. Immunol.* **9:**271-296). For example, these cells include lymphoid dendritic cells of the spleen, Langerhans cells of the epidermis, and veiled cells in the blood circulation. Dendritic cells are collectively classified as a group based on their morphology, high levels of surface MHC-class II expression as well as expression of several accessory molecules (B7-1[CD80] and B7-2[CD86]) that mediate **T** cell binding and costimulation (Inaba, *et al.,* 1990, *Intern. Rev. Immunol.* **6**:197-206; Frendenthal, *et al.,* 1990, *Proc. Natl. Acad. Sci. USA* **87**:7698). In addition, the absence of certain other surface markers expressed on T cells, B cells, monocytes, and natural killer cells is indicative of dendritic cells.

Dendritic cells are bone marrow-derived and migrate as precursors through blood stream to tissues, where they become resident cells such as Langerhans cells in the epidermis. In the periphery, following pathogen invasion, immature dendritic cells such as fresh Langerhans cells are recruited at the site of inflammation (Kaplan, *et al.*, 1992, *J. Exp. Med.* **175**:1717-1728; McWilliam, *et al.,* 1994, *J. Exp. Med.* **179**:1331-1336) where they capture and process antigens (Inaba, *et al.,* 1986. *J. Exp. Med.* **164**:605-613; Streilein, *et al.,* 1989, *J. Immunol.* **143**:3925-3933; Romani, *et al.,* 1989., *J. Exp. Med.* **169**:1169-1178; Puré, *et al.,* 1990. *J. Exp. Med.* **172**:1459-1469; Schuler, *et al.,* 1985, *J. Exp. Med.* **161**:526-546).

Antigen-loaded dendritic cells then migrate from the peripheral tissue via the lymphatics to the T cell rich area of lymph nodes, where the mature dendritic cells are called interdigitating cells. (Austyn, *et al.,* 1988, *J. Exp. Med.* **167**:646-651; Kupiec-Weglinski, *et al.,* 1988, *J. Exp. Med.* **167**:632-645; Larsen, *et al.,* 1990, *J. Exp. Med.* **172**:1483-1494; Fossum, S. 1988, *Scand. J. Immunol.* **27**:97-105; Macatonia, *et al.,* 1987, *J. Exp. Med.* **166**:1654-1667; Kripke, *et al.,* 1990., *J. Immunol.* **145**:2833-2838). At this site, they present the processed antigens to naive T cells and generate an antigen-specific primary T cell response (Liu, *et al.,* 1993, *J. Exp. Med.* **177**:1299-1307; Sornasse, *et al.*, 1992, *J. Exp. Med.* **175**:15-21; Heufler, *et al.,* 1988. , *J. Exp. Med.* **167**:700-705).

During their migration from peripheral tissues to lymphoid organs, dendritic cells undergo a maturation process encompassing dramatic changes in phenotype and functions (Larsen, *et al.*, 1990, *J. Exp. Med.* **172**:1483-1494; Streilein, *et al.*, 1990, *Immunol. Rev.* **117**:159-184; De Smedt, *et al.,* 1996, *J. Exp. Med.* **184**:1413-1424). In particular, in contrast to immature dendritic cells such as fresh Langerhans cells, which capture and process soluble proteins efficiently and are effective at activating specific memory and effector T cells, mature dendritic cells such as interdigitating cells of lymphoid organs are poor in antigen capture and processing but markedly efficient in naive T cell priming (Inaba, *et al.*, 1986. *J. Exp. Med.* **164**:605-613; Streilein, *et al.*, 1989, *J. Immunol.* **143**:3925-3933; Romani, *et al.*, 1989, *J. Exp. Med.* **169**:1169-1178; Puré, *et al.*, 1990, *J. Exp. Med.* **172**:1459-1469; Sallusto, *et al.*, *1995, J. Exp. Med.* **182**:389-400; Cella, *et al.*, 1997, *Current Opin Immunol.* **9:**10-16). The signals regulating complex trafficking and maturation patterns of dendritic cells are complex and not fully understood.

In addition to their function in antigen presentation and activation of T cells, tumor antigen loaded dendritic cells have been shown to prevent the development of tumors and even to induce the regression of established tumors. Moreover, there is now evidence that certain subpopulations of dendritic cells may induce a state of tolerance, a property that may prove very useful in the field of allotransplantation and possibly, xenotransplantation. It is thus critical to define which dendritic cell subset should be targeted in different pathologies.

Despite the importance of dendritic cells to immune system function and related tumor growth suppression, dendritic cells remain poorly characterized, in terms of their origin, in terms of the proteins they express and in terms of many of their functions. In particular, the processes and mechanisms related to the initiation of an immune response, including antigen processing and presentation are not well elucidated.

Dendritic cells originate from several sources including myeloid and non-myeloid derived cell types. Many of the dendritic cells identified thus far derive from monocyte (myeloid) origin. A heretofore-unclassified subset of dendritic cells putatively derived from lymphoid origin is particularly worthy of study and classification because this subset of dendritic cells produces vast amounts of interferon-α. Interferon-α is especially important in the supression of viral pathogens and has also been implicated in cancer suppression.

There is thus a critical need in the art for the identification of genes and proteins involved in dendritic cell maturation, trafficking and function. In addition, there is a need for agents useful in the diagnosis and treatment of medical conditions caused by the inappropriate regulation, development, and/or physiology of these cells which are so crucial to immune system function.

### Summary of The Invention

The present invention fulfills these needs by providing novel genes isolated from dendritic cells. This particular subset of dendritic cells are of putative lymphoid versus myeloid origin. The novel genes likely encode such molecules as cell surface receptors and secretory proteins. These genes can be used to study the presence, amount, distribution and normalcy of certain gene products produced by or expressed on dendritic cells. They can also be used to facilitate the discovery of compositions and methods useful for diagnosing and treating certain disease states.

In one embodiment, the invention provides isolated polypeptides comprising amino acid sequences derived from SEQ ID NOS: 2, 4 or 6. Polypeptides derived from SEQ ID NOS: 2, 4 or 6 comprise at least eight, preferably at least ten, and most preferably, at least about twelve or more consecutive amino acid residues from SEQ ID NOS: 2, 4 or 6 as well as sequence-and-function conservative variants of those sequences. In a preferred embodiment, the amino acid sequences encode for a mature protein.

In a related embodiment, the invention provides isolated polypeptides comprising nucleic acid sequences encoding amino acid sequences derived from SEQ ID NOS: 2, 4 or 6. The isolated nucleic acid sequences comprise at least about 12, preferably, at least about 18, more preferably, at least about 20-35, and most preferably, 35-55 or more consecutive nucleotides from SEQ ID NOS: 2, 4 or 6. The nucleic acids may be DNA, RNA, DNA/RNA duplexes, protein-nucleic acids or derivatives thereof. In a preferred embodiment, the nucleic acid sequences comprise the nucleic acid sequences set forth in SEQ ID NOS: 1, 3 or 5.

The invention also encompasses recombinant DNA vectors, including DNA and expression vectors, comprising the nucleotide sequences of the invention; cells comprising such vectors, including bacterial, fungal, plant, insect, and mammalian cells and methods for producing expression products comprising RNA and polypeptides encoded by the sequences.

In still another embodiment, the invention provides a binding compound which specifically binds to the polypeptide of claim 1. Preferably, that binding compound is an antibody or antibody fragment. Most preferably, the binding compound is a monoclonal antibody.

The invention also provides methods for detecting the nucleic acids and polypeptides of the invention in a sample. A method for detecting nucleic acids of the invention comprises the steps of: (1) contacting the sample with a probe comprising a nucleic acid comprising at least eight consecutive nucleotides selected from SEQ ID NOS: 1, 3 or 5 under conditions where hybridization can occur; and (2) detecting hybridization, if any. A method for detecting the polypeptides of the invention comprises the steps of: (1) contacting the sample with an antibody or antibody fragment specific for the polypeptide to be detected; and (2) detecting the presence of an antigen-antibody complex.

Finally, the invention provides a method of screening for candidate therapeutic agents comprising the steps of: (1) selecting as a target a polypeptide having an amino acid sequence derived from SEQ ID NOS: 2, 4 or 6; and (2) contacting a test compound with the target sequence; and (3) selecting as candidate therapeutic agents test compounds which bind to the target sequence.

### Detailed Description of the Invention

The present invention relates to nucleic acids and encoded proteins from dendritic cells. In addition, the invention relates to diagnostic and therapeutic methods utilizing these nucleic acids and proteins.

All patent applications, patents, and literature references cited in this specification are hereby incorporated by reference in their entirety.

In practicing the present invention, many conventional techniques in molecular biology, microbiology, and recombinant DNA, are used. Such techniques are well known and are explained fully in, for example, Sambrook *et al.,* 1989, *Molecular Cloning: A Laboratory Manual,* Second Edition, Cold Spring Harbor Laboratory Press, Cold Spring Harbor, New York; *DNA Cloning: A Practical Approach,* Volumes I and II, 1985 (D.N. Glover ed.); *Oligonucleotide Synthesis,* 1984, (M.L. Gait ed.); *Nucleic Acid Hybridization,* 1985, (Hames and Higgins); *Transcription and Translation,* 1984 (Hames and Higgins eds.); Animal Cell Culture, 1986 (R.I. Freshney ed.); *Immobilized Cells and Enzymes,* 1986 (IRL Press); Perbal, 1984, *A Practical Guide to Molecular Cloning*; the series, *Methods in Enzymology* (Academic Press, Inc.); *Gene Transfer Vectors for Mammalian Cells,* 1987 (J. H. Miller and M. P. Calos eds., Cold Spring Harbor Laboratory); and *Methods in Enzymology* Vol. 154 and Vol. 155 (Wu and Grossman, and Wu, eds., respectively).

Before describing the invention in detail, the following definitions are provided to aid in an understanding of the specification and claims:

A "dendritic-derived" nucleic acid or polypeptide refers to the source from which the sequence was originally isolated.

An "enriched gene product" is a protein produced in copious amounts by a particular cell type or tissue.

A "Nucleic acid" or "polynucleotide" refers to purine- and pyrimidine-containing polymers of any length, either polyribonucleotides or polydeoxyribonucleotides or mixed polyribo-polydeoxyribo nucleotides. This includes single- and double-stranded molecules, i.e., DNA-DNA, DNA-RNA and RNA-RNA hybrids, as well as "protein nucleic acids" (PNA) formed by conjugating bases to an amino acid backbone. This also includes nucleic acids containing modified bases.

A "coding sequence" or a "protein-coding sequence" is a polynucleotide sequence capable of being transcribed into mRNA and/or capable of being translated into a polypeptide. The boundaries of the coding sequence are typically determined by a translation start codon at the 5'-terminus and a translation stop codon at the 3'-terminus.

A "complement" of a nucleic acid sequence refers to the "antisense" sequence that participates in Watson-Crick base-pairing with the original sequence.

An "isolated" nucleic acid or polypeptide refers to a component that is removed from its original environment (for example, its natural environment if it is naturally occurring). An isolated nucleic acid or polypeptide preferably contains less than about 50%, more preferably less than about 75%, and most preferably less than about 90%, of the cellular components with which it was originally associated.

A nucleic acid or polypeptide sequence that is "derived from" a designated sequence refers to a sequence that corresponds to a region of the designated sequence. For nucleic acid sequences, this encompasses sequences that are homologous or complementary to the sequence, as well as "sequence-conservative variants" and "function-conservative variants." For polypeptide sequences, this encompasses "function-conservative variants." Sequence-conservative variants are those in which a change of one or more nucleotides in a given codon position results in no alteration in the amino acid encoded at that position. Function-conservative variants are those in which a given amino acid residue in a polypeptide has been changed without substantially altering the overall conformation and function of the native polypeptide, including, but not limited to, replacement of an amino acid with one having similar physico-chemical properties (such as, for example, acidic, basic, hydrophobic, and the like). "Function-conservative" variants also include any polypeptides that have the ability to elicit antibodies specific to a designated polypeptide.

A "probe" refers to a nucleic acid or oligonucleotide that forms a hybrid structure with a sequence in a target region due to complementarity of at least one sequence in the probe with a sequence in the target.

Nucleic acids are "hybridizable" to each other when at least one strand of nucleic acid can anneal to another nucleic acid strand under defined stringency conditions. Stringency of hybridization is determined, e.g., by a) the temperature at which hybridization and/or washing is performed, and b) the ionic strength and polarity (e.g., formamide) of the hybridization and washing solutions, as well as other parameters. Hybridization requires that the two nucleic acids contain substantially complementary sequences; depending on the stringency of hybridization, however, mismatches may be tolerated. The appropriate stringency for hybridizing nucleic acids depends on the length of the nucleic acids and the degree of complementarity, variables well known in the art.

An "immunogenic component" is a moiety that is capable of eliciting a humoral and/or cellular immune response in a host animal.

An "antigenic component" is a moiety that binds to its specific antibody with sufficiently high affinity to form a detectable antigen-antibody complex.

A "sample" refers to a biological sample, such as, for example, tissue or fluid isolated from an individual or from *in vitro* cell culture constituents, as well as samples obtained from laboratory procedures.

The invention provides nucleic acid sequences encoding mammalian proteins expressed by and/or on dendritic cells. While specific human dendritic cell-derived genes and gene products are described herein, the invention encompasses structurally (e.g., sequence) related embodiments from other sources or mammalian species, including polymorphic or individual variants. These will include, e.g., proteins which exhibit relatively few changes in sequence, e.g., less than about 5%, and number, e.g., less than 20 residue substitutions, typically less than 15, preferably less than 10, and more preferably less than 5 substitutions. These will also include versions which are truncated from full length and fusion proteins containing substantial segments of these sequences.

The present inventors have isolated three genes from dendritic cells of putative lymphoid origin. Humans have two distinct subsets of dendritic cell precursors. Peripheral blood monocytes give rise to mature myeloid (type I) dendritic cells (DC1) after stimulation with several co-factors and/or appropriate processing in the body. Type II dendritic cells (DC2) arise from putative lymphoid origin. For example, plasmacytoid cells (lymphoid) from human tonsils and blood differentiate into type II dendritic cells. (Rissoan, *et al.,* 1999, Science **283**:1183). Type I and type II dendritic cells have different surface markers. Type II, CD3⁻CD4⁺CD11c⁻ dendritic cells are the main interferon-α producers in response to enveloped viruses, bacteria and tumor cells. (Siegal, *et al.,* 1999, Science **284**:1835). Example I describes the isolation of these type II dendritic cells.

### EXAMPLE I

### Purification of CD3⁻CD4⁺CD11c⁻Lin⁻Cells

CD3⁻CD4⁺CD11c⁻ cells were isolated from human tonsils. In brief, tonsils were cut into small pieces and digested for 12 min at 37°C with collagenase IV (1 mg/ml; Sigma) and deoxyribonuclease I (50 KU/ml, Sigma) in RPMI 1640. The cells, pooled from two rounds of tissue digestion, were centrifuged over 50% Percoll (Pharmacia Uppsala, Sweden) for 20 min at 400 g. CD3⁺ T cells, CD14⁺ monocytes, CD19⁺ and CD20⁺ B cells, and CD56⁺ NK cells were depleted from the resulting low density cells by immunomagnetic beads (sheet anti-mouse Ig-coated Dynabeads; Dynal, Oslo, Norway). The resulting cells were stained with mouse anti-CD4-PE-Cy5 (Immunotech), anti-CD11c-PE (Becton Dickinson), and a cocktail of FITC-labeled mAbs anti-CD3 and anti-CD34 (Immunotech), anti-CD20, anti-CD57, anti-CD7, anti-CD14, and anti-CD16 (Becton Dickinson), and anti-CD1a (Ortho). Then CD3⁻CD4+CD11c-Lin⁻Cells were isolated by cell sorting. Reanalysis of the sorted cells confirmed a purity of 98%. (Grouard *et al.,* 1997, *J. Exp. Med.,* **185**:1101-1111)

Three genes (hereinafter referred to as contigs 58, 92, and 20) produced primarily and/or exclusively by CD3⁻CD4+CD11c- cells were isolated, sequenced, and functionally identified via cDNA subtraction. Contig 58 has a nucleotide sequence set forth in SEQ ID NO: 1 and an amino acid sequence set forth in SEQ ID NO: 2. Contig 92 has a nucleotide sequence set forth in SEQ ID NO: 3 and an amino acid sequence set forth in SEQ ID NO: 4. Contig 20 has a nucleotide sequence set forth in SEQ ID NO: 5 and an amino acid sequence set forth in SEQ ID NO: 6. Briefly, cDNA subtraction is a method for finding genes expressed in one mRNA population but reduced or absent in another. Example II describes the subtraction process.

### EXAMPLE II

### Construction of subtracted cDNA library

cDNA made from mRNA isolated from 98% purified CD3⁻CD4+CD11c⁻ cells from tonsils was subtracted from cDNA made from mRNA isolated from monocyte-derived DC1s in order to determine gene products produced exclusively, or at least, showing enriched production by CD3⁻CD4⁺CD11c⁻ cells. The CD3⁻CD4⁺CD11c⁻ cells were cultured in IL-3 and CD40L fibroblasts overnight or 48 hours (the two subsets of cells were pooled). The monocyte-derived DC1s were cultured 6 days in GM-CSF and IL4, activated with GM-CSF+CD40L overnight or for 48 hours. Complimentary DNA synthesis and subtration was done utilizing the PCR-select kit (Clontech, Palo Alto, CA) using Advantage™ Klen Tag polymerase (Clontech). The first PCR reaction was done for 28 cycles and the second (nested) PCR for 12 cycles on a thermal cycler (480; Perkin-Elmer Corp., Norwalk, CT). To clone subtracted DC cDNA, 10 nested reactions were pooled and resolved on 2% low melting agarose. Aiming for individual bands, gel slices in the 0.7-1.4 kb size range were cut out; the DNA was eluted and cloned either directly or after reamplification into a T/A-vector (pCRII Invitrogen Corp., San Diego, CA). The inserts were sequenced in both directions by automatic sequencing. Comparisons against GenBank and dbest databases as well as protein homology prediction were obtained from the NCBI blast server (http://www.ncbi.nlm.nih.gov/BLAST/).

### Contig 58

Contig 58 is an enriched gene product of the plasmacytoid cells described in Example I, but is not exclusively produced by these cells. Contig 58 is encoded by SEQ ID NO: 1. The amino acid sequence of the protein encoded by contig 58 is shown in SEQ ID NO: 2.

After obtaining the complete sequence of contig 58, BLAST analysis was used to analyze the sequence in terms of homology to other genes. Example III describes this process.

### EXAMPLE III

### Sequence Comparison of Isolated Contigs to Known Genes

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optical alignment of sequences for comparison can be conducted, e.g., by the local homology algorithm of Smith and Waterman (1981) Adv. Appl. Math. **2**:482, by the homology alignment algorithm of Needleman and Wunsch (1970) J. Mol. Biol. **48**:443, by the search for similarity method of Pearson and Lipman (1988) Proc. Nat'l Acad. Sci. USA **85**:2444, by computerized implementations of these algorithms (GAP, BESTFIT, FASTA, and TFASTA in the Wisconsin Genetics Software Package, Genetics Computer Group, 575 Science Dr., Madison, WI), or by visual inspection (see generally Ausubel et al., supra).

An example of an algorithm that is suitable for determining percent sequence identity and sequence similarity is the BLAST algorithm, which is described in Altschul, et al. (1990) J. Mol. Biol. **215**:403-410. Software for performing BLAST analyses is publicly available through the National Center for Biotechnology Information (http:www.ncbi.mm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul, et al., supra). These initial neighborhood word hits act as seeds for initiating searches to find longer HSPs containing them. The word hits are then extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff and Henikoff (1989) Proc. Nat'l Acad. Sci. USA **89**:10915) alignments (B) of 50, expectation (E) of 10, M=5, N=4, and a comparison of both strands.

In addition to calculating percent sequence identity, the BLAST algorithm also performs a statistical analysis of the similarity between two sequences (see, e.g., Karlin and Altschul (1993) Proc. Nat'l Acad. Sci. USA **90**:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Conserved alignment patterns, discerned at several degrees of stringency, were drawn by the consensus program (internet URL http://www.bork.embl-Leidelberg.de/Alignment/consensus.html). The PRINTS library of protein fingerprints (http://www.biochem.ucl.ac.uk/bsm/dbbrowser/PRINTS/PRINTS/html) (Attwood, et al. (1997) *Nucleic Acids Res.* 25:212-217) reliably identified the leucine-rich repeats (LRRs) present in the extracellular segment of contig 58 with a compound motif (PRINTS code Leurichrpt) that flexibly matches N- and C-terminal features of divergent LRRs. Two prediction algorithms whose three-state accuracy is above 72% were used to derive a consensus secondary structure for the intracellular domain alignment, as a bridge to fold recognition efforts (Fischer, et al. (1996) *FASEB J.* 10:126-136). Both the neural network program PHD (Rost and Sander (1994) *Proteins* 19:55-72) and the statistical prediction method DSC (King and Sternberg (1996) *Protein Sci.* 5:2298-2310) have internet servers (URLs http://www.embl-eidelberg.de/predictprotein/phd_pred.html and http:bonsai.lif.icnet.uk/bmm/dsc/dsc_read_align.html, respectively).

### EXAMPLE IV

### Chromosomal localization of contigs

Chromosomal localization was performed with the Stanford G3 RH medium resolution panel (Research Genetics, Huntsville, AL, U.S.A.). PCR is performed using oligonucleotides which amplify specifically the gene of interest and which do not amplify the mouse equivalent. Results are scored by the presence or absence of a PCR fragment in genomic DNA from the different cell lines. This information is scored manually and analysis is performed using the RH mapper program (http://shgc-www.stanford.edu). Using this method, contig 58 has been localized to chromosome 19q13-q12, a region of the chromosome containing many genes encoding cell surface receptors of the immune system. Equally, by homology to the BAC clone AC008397, a similar chromosomal localization is indicated.

In addition to BLAST analysis, the predicted ORF of contig 58 was analyzed with respect to motifs and protein localization signals using pSORT.

### EXAMPLE V

### pSORT Analysis of Contigs

### 1. Recognition of Signal Sequences

In eukaryotes, proteins sorted through the so-called vesicular pathway (bulk flow) usually have a signal sequence (also called a leader peptide) in the N-terminus, which is cleaved off after the translocation through the ER membrane. Some N-terminal signal sequences are not cleaved off, remaining as transmembrane segments but it does not mean these proteins are retained in the ER; they can be further sorted including in vesicles. pSORT first predicts the presence of signal sequences by McGeoch's method (D. J. McGeoch, Virus Res., **3**:271, 1985) modified by Nakai and Kanehisa, *(Proteins* 11**(2)**:95-110 1991) and Nakai, 1996. It considers the N-terminal positively-charged region (N-region) and the central hydrophobic region (H-region) of signal sequences. A discriminant score is calculated from the three values: length of H-region, peak value of H-region, and net charge of N-region. A large positive discriminant score means that there is a high possibility that the protein possess a signal sequence, but it is unrelated to the possibility of its cleavage. Next, pSORT applies von Heijne's method of signal sequence recognition (G. von Heijne, Nucl. Acids Res., **14**:4683, 1986). It is a weight-matrix method and incorporates the information of consensus pattern around the cleavage sites (the (-3,-1)-rule) as well as the feature of the H-region. Thus it can be used to detect signal-anchor sequences. The output score of this "GvH" is the original weight-matrix score (for eukaryotes) subtracted by 3.5. A large positive output means a high possibility that it has a cleavable signal sequence. The position of possible cleavage site, i.e., the most C-terminal position of a signal sequence, is also reported.

### 2. Recognition of Transmembrane Segments

The current version of pSORT assumes that all integral membrane proteins have hydrophobic transmembrane segment(s) which are thought to be alpha-helices in membranes. pSORT employs Klein et al.'s method (ALOM, also called KKD) to detect potential transmembrane segments ( P. Klein, M. Kanehisa, and C. DeLisi, Biochim. Biophys. Acta, **815:**468, 1985) modified by Nakai and Kanehisa, *(Genomics,* 1992, 14**(4)**:897-911 1992). It repeats to identify the most probable transmembrane segment from the average hydrophobicity value of 17-residue segments, if any. It predicts whether that segment is a transmembrane segment (INTEGRAL) or not (PERIPHERAL) comparing the discriminant score (reported as 'ALOM score') with a threshold parameter. For an integral membrane protein, the position(s) of transmembrane segment(s) are also reported. Their length is fixed to 17 but their extension, i.e., the maximal range that satisfies the discriminant criterion, is also given in parentheses. The discrimination step mentioned above is continued after leaving out the detected segment until there remains no predicted transmembrane segment. The item 'number of TMSs' is the number of predicted transmembrane segments. Since this algorithm is applied to a predicted mature sequence, i.e., cleavable signal sequence is not included, this number is expected to be the one for mature proteins. The modification by Nakai and Kanehisa, 1992 was to employ two kinds of threshold values because ALOM is not very accurate to predict the exact number of transmembrane segments of polytopic, i.e., multiple membrane-spanning, proteins. The rationale of the approach is that less hydrophobic segments are likely to be more easily integrated into the membrane once a part of the polypeptide is integrated. Specifically, pSORT first tentatively evaluates the number of transmembrane segments using less stringent value (0.5). Then, it re-evaluates the number by using a more stringent threshold (-2.0). If it is still predicted to have at least one transmembrane segment, the former threshold value is used.

### 3. Prediction of Membrane Topology

Every membrane protein has its own orientation to be integrated in the membrane. In other words, membrane proteins know at which side (cytoplasmic or exo-cytoplasmic) the N-terminus should be located. Such an orientation is called the membrane topology. Singer's classification for membrane topology ( S. J. Singer, Ann. Rev. Cell Biol., **6**:247, 1990) was utilized. Prediction of membrane topology is important because some sorting signals exist at specific positions, e.g., the cytoplasmic tail, in a certain topology (see below). pSORT uses Hartmann et al.'s method (E. Hartmann, T. A. Rapoport, and H. F. Lodish, Proc. Natl. Acad. Sci. USA, **86**:5786, 1989); called "MTOP" in pSORT) for the prediction of membrane topology. MTOP assumes that the overall topology of eukaryotic membrane proteins is determined by the net charge difference of 15 residues flanking the most N-terminal transmembrane segment on both sides. The central residue of such a segment is first reported. If a protein is predicted to have a cleavable signal sequence and one transmembrane segment, its topology is '1a'. If a protein is predicted to have no cleavable signal sequence but has one transmembrane segment, its position is examined. If it exists near its C-terminus. Its topology is assigned as 'Nt (N-tail)' (see U. Kutay et al., Trends Cell Biol., **3**:72-75, 1993). Otherwise, its topology is assigned to '1b' or '2' depending on the charge difference reported by MTOP. For polytopic proteins, their topology is simply predicted by MTOP.

For contig 58, pSORT predicts a type 1 membrane protein i.e. a protein with a cleavable signal peptide on the N-terminal (from amino acids approximately 1-24) and a single transmembrane segment from approximately 166-182. The N-terminal end of the protein is outside the cell and the C-terminal end is inside the cell. The protein encoded by contig 58 is characterized as having several leucine-rich repeats. These repeats are often associated with protein/protein interactions. A wide range of potential ligands recognize these motifs, including members of the cysteine knot family of small, secreted cytokine-like molecules. This type of domain is also known to interact with non-protein ligands such as nucleic acid or lipopolysaccaride. Thus, the molecule encoded by contig 58 may represent a novel pattern-recognition receptor.

The predicted amino acid sequence also shows two tyrosine-based motifs, one for interaction with PI3 kinase (YENM), and an ITAM (immunoreceptor tyrosine-based activation motif: YXXI/L X(7/8) YXXI/L). These motifs are described in the following references: Biery M. Olcese *et al.,* "Early Signaling via Inhibitory and Activating NK Receptors," *Hum Immunol* **1**:51-64 (Jan. 6, 2000); Gergely J. Pecht I *et al.,* "Immunoreceptor tyrosine-based inhibition motif-bearing Receptors Regulate the Immunoreceptor Tyrosine-based activation motif-induced activation of Immune Competent Cells," Immunol Lett **1**:3-15 (May 1999); Daeron, M., "Structural Bases of Fc Gamma R functions," *Int. Rev. Immunol* **16**:1-27, (1997).

Contig 58 is believed to be a type of molecule which is a component of membrane complexes which, when cross-linked give signals that lead to cell activation. The ITAM type of motif is generally associated with immunoglobulin superfamily members or lectins, thus this molecule may represent a novel family of cell surface receptors.

Based on its structure as elucidated by pSORT, its homology to other genes as elucidated by BLAST, and its amino acid sequence, contig 58 might represent a novel type of receptor involved in the danger-induced activation of the innate and acquired immune cells. Triggering the receptor encoded by contig 58 might stimulate or modulate immune function. Equally, blocking of this receptor might allow to reduce the effects of overstimulation in conditions such as auto-immunity, graft rejection, and toxic shock.

### EXAMPLE VI

### Expression analysis by RT-PCR

The expression pattern of contig 58 was analyzed by Reverse Transcriptase Polymerase chain reaction (RT-PCR). mRNAs were isolated from cells, tissues or cell lines using oligo dT-coupled magnetic beads to isolate the polyA containing mRNAs and first strand cDNA synthesis was carried out using standard methods. The expression pattern of contig 58 was then analyzed. On freshly isolated cells contig 58 is expressed by non-activated B cells, monocytes and CD3-CD4+Cd11c-DC. On cells generated *in vitro,* contig 58 is expressed by granulocytes and DC generated from CD34+ progenitors. Expression of the gene encoded by contig 58 is not seen in cell lines such as MRC5 (fetal lung fibroblast), CHA (kidney epithelial cell) JY (EBV-derived B cell line), Jurkat (T cell line), U937 (histiocytic myeloma) and TF1 (hematopoietic progenitor).

This new molecule encoded by contig 58 is down-regulated by CD40-ligand activation in hematopoietic cells. No mRNA was detected in PMA-Ionomycin activated hematopoietic and non-hematopoietic cell lines.

### EXAMPLE VII

### Expression analysis by Tissue Northern Blot

Total RNA was used for the preparation of Northern blots and first strand cDNA. Northern blots were prepared using 10 µg total RNA separated on formaldehyde denaturing gels and blotted onto Hybond N⁺ membranes (Amersham, Les Ulis, France). Human adult and fetal tissue blots were also used (MTN blots 7760-1 and 7756-1 Clontech, Palo Alto, CA.). Genomic DNA was isolated from PBL using standard techniques, cut with restriction enzymes, separated on 1% agarose gels and blotted onto Hybond N⁺ membranes. Hybridization of Northern and Southern blots was with a DNA fragment corresponding to a specific cDNA labeled with [³²P]dCTP using the High Prime Kit (Boehringer Mannheim, Meylan, France). High stringency washes were performed using 0.2 x SSC, 0.2% SDS twice for 30 min. First strand cDNA were prepared after Dnase I treatment of 5 µg total RNA using oligo (dT) primers (Pharmacia, Orsay, France) using the Superscript kit. Synthesis of cDNA was checked by RT-PCR using β-actin primers. RT-PCR was performed using the AmpliTaq enzyme and buffer (Perkin Elmer, Paris, France), 0.8 mM dNTP and DMSO at 5% final concentration.

The Northern-blot shows that contig 58 exhibits a strong expression in peripheral blood lymphocytes and a lower expression in the spleen and the lymph nodes. There is no detectable expression in cancer cell lines at 60 hours of exposition. After 18 days, there is weak expression in Burkitt's lymphoma Raji (EBV B cell line).

### Contig 92

Contig 92 appears to be mainly produced by the plasmacytoid cells described in Example I. For contig 92, pSORT predicts a type II transmembrane protein with an uncleaved signal anchor sequence from approximately 57-74. The topology predicted for this protein is C-terminal extracellular, N-terminal cytosolic.

### Expression analysis by RT-PCR:

See Example VI for a detailed protocol utilized to analyze the expression patterns of the protein encoded by contig 92. The expression pattern of contig 92 was analyzed by RT-PCR. This gene was extremely strongly expressed by CD3- CD4+. CD11c- DC, expressed by monocytes and DC derived from monocytes, weakly expressed in *in vitro* generated granulocytes and very weakly expressed in T, B cells, and the cell lines JY and Jurkat.

### Expression analysis by Tissue Northern Blot:

See Example VII for a detailed protocol. Expression of the protein encoded by contig 92 is detected in all hematopoietic cells, very strongly in thymus and appendix, strongly in lymph node and spleen, fetal liver, bone marrow and more weakly on peripheral blood lymphocytes. In cancer cell lines, expression is found in melanoma, and in chronic leukemia MOLT-4.

### Contig 20

For contig 20, pSORT predicts a secreted protein with a cleavable signal peptide.

### Expression analysis by RT-PCR:

See Example VI for a detailed protocol. The expression pattern of contig 20 was analyzed by RT-PCR. This gene is expressed by freshly-isolated T and B cells, CD3- CD4+ Cd11c- DC, *in vitro* generated granulocytes and DC generated from CD34+ progenitors, and monocytes. In addition, it is expressed by the hematopoetic cell lines JY and Jurkat, but not U937 and TF1, and not in the non-hematopoetic cell lines CHA and MRC5.

### Expression analysis by Tissue Northern Blot (Clontech):

See Example VII for a detailed protocol. The Northern blot shows one major band at 0,7 kb and two bands with higher molecular weights of 4, 4 and 7, 5kb.

The 0,7 kb band corresponds to the predicted cDNA sequence shown in SEQ ID NO: 6. The two bands of higher MW are probably the nuclear pre-mRNA (non-spliced). This new protein, contig 20, is highly expressed in human bone marrow, peripheral blood lymphocytes, spleen, and lymph node. In thymus, appendix and fetal liver the expression is reduced. Contig 20 is expressed in the human cancer cell lines of Burkitt's lymphoma Raji and chronic leukemia MOLT-4.

Contig 20 likely encodes a putative secreted molecule which is expressed by hematopoietic cells. The protein expressed in contig 20 might be a cytokine, because it is secreted, it is in hematopoietic cells, and its large distribution suggests that the protein is important in cellular interactions.

The protein encoded by contig 20 may also be a defensin-like molecule because defensins are a family of small peptides with three or four intramolecular cysteine disulfide bonds, and defensins have antimicrobial activity based on their capacity to chemoattract immune cells, thereby promoting host immunity.

Contig 20 might represent a novel broad range hematopoietic growth factor, and autocrine factor for some cancer (leukemias). It may control both quantitatively and qualitatively the immune cells interactions and could be used as an immunostimulant or as an immunomodulator. Alternatively, contig 20 might represent a target for blocking agents in conditions of unwanted immune reactivity such as auto-immune diseases or graft rejection.

### Mouse equivalents

The mouse homologs of the sequences described above were detected by using BLAST analysis (tBLASTn) of the ORF of each gene to detect mouse ESTs that potentially code for a protein with identical characteristics. Contigs were made with the resulting sequences and the predicted protein was analyzed as per the human proteins.

The mouse homolog of contig 58 is coded for by ESTs W85307 and AI430301.

The mouse homology of contig 92 is expressed in brain, ESTAB030199. **[need cite?]**

Multiple ESTs, over 80, were identified as mouse homologs to contig 20. The most 5' of thsese (and those containing in principle all the ORF) are AA968242, AA796464, AI317775. The northern blot shows very strong expression in spleen, strong expression in heart, lung and liver and weak expression in kidney at a size of 2 kb. In testis, 2 bands are seen of 2 and 2.4 kb. The 2.4kb band is stronger. No expression is seen in skeletal muscle and brain.

### Nucleic Acids, Vectors, and Host Cells

The invention provides nucleic acid sequences, in particular the nucleic acid sequences shown in SEQ ID NOS: 1, 3 or 5 or nucleic acid sequence which encode an amino acid sequences shown in SEQ ID NOS: 2, 4 or 6. The invention encompasses isolated nucleic acid fragments comprising all or part of the individual nucleic acid sequences disclosed herein. The nucleic acid sequences of the invention comprise at least about 12, preferably at least about 18, more preferably at least about 20-35 and most preferably at least about 35-55 or more consecutive nucleotides, including complete protein-coding sequences, or complements thereof. The invention encompasses sequence-conservative variants and function-conservative variants of these sequences.

Nucleic acids comprising any of the sequences disclosed herein or subsequences thereof can be prepared by standard methods using the nucleic acid sequence information provided in SEQ ID NOS: 1, 3 or 5. For example, nucleic acids can be chemically synthesized using, e.g., the phosphoramidite solid support method of Matteucci *et al.,* 1981, *J. Am. Chem. Soc.* **103**:3185, the method of Yoo *et al.,* 1989, *J.* *Biol. Chem.* **764**:17078, or other well known methods. This can be done by sequentially linking a series of oligonucleotide cassettes comprising pairs of synthetic oligonucleotides. The nucleic acids may be isolated directly from cells. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression. Of course, due to the degeneracy of the genetic code, many different nucleotide sequences can encode polypeptides having the amino acid sequences defined by SEQ ID NOS: 2, 4 or 6 subsequences thereof. The codons can be selected for optimal expression in prokaryotic or eukaryotic systems. Such degenerate variants are also encompassed by this invention.

The encoded polypeptides may be expressed by using many known vectors such as pUC plasmids, pET plasmids (Novagen, Inc., Madison, WI), or pRSET or pREP (Invitrogen, San Diego, CA), and many appropriate host cells such as *Escherichia coli, Saccharomyces cerevisiae*, and insect and mammalian cell lines using methods known to those skilled in the art. The particular choice of vector/host is not critical to the practice of the invention.

The nucleic acids of the present invention find use, e.g., as templates for the recombinant production of peptides or polypeptides, as probes and primers for the detection of the human genes described herein, for chromosome mapping, and as probes or to design PCR primers to identify homologous genes in other mammalian species. Homology may be determined experimentally. Alternatively, homology analysis may be performed computationally. In practicing the present invention, a gene that shares at least about 70% DNA sequence homology at the nucleotide level with the genome of another mammalian species is considered to be present in that species. The determination that a gene is present in another mammal may be achieved using any technique known in the art. Appropriate techniques include without limitation hybridization to genomic DNA, colony hybridization to a genomic or cDNA library, polymerase chain reaction (PCR) using degenerate primers or gene-specific primers and genomic DNA as a template, genetic complementation, antibody cross-reactivity, or biochemical complementation *in vitro.*

In applying these techniques, conditions are established that discriminate different levels of homology between probe and template. For example, for hybridization of a probe to immobilized DNA (whether in a Southern blot, dot blot, or colony hybridization format), varying the SSC concentration in the buffer allows the detection of hybrids having different levels of homology (1X SSC is 0.15 M NaCl-0.015 M Na citrate). In a wash buffer containing 6M urea and 0.4% sodium dodecyl sulfate, the presence of 2X SSC, 0.5X SSC, 0.1X SSC, and 0.05X SSC allows the formation of hybrids having threshold homologies of at least 55% + 5%, 65% + 5%, 75% + 5%, and >85%, respectively. Preferably, once a gene has been identified in another organism by hybridization or PCR, the DNA sequence of the gene is determined directly.

It will be understood that some methods that detect homologous sequences may result in the identification or isolation of only a portion of the entire protein-coding sequence of a particular gene. The entire protein-coding sequence can be isolated and identified, for example, by using an isolated nucleic acid encoding the known portion of the sequence, or fragments thereof, to prime a sequencing reaction with cDNA as template; this is followed by sequencing the amplified product. The isolated nucleic acid encoding the disclosed sequence, or fragments thereof, can also be hybridized to appropriate cDNA libraries to identify clones containing additional complete segments of the protein-coding sequence of which the shorter sequence forms a part. Then, the entire protein-coding sequence, or fragments thereof, or nucleic acids encoding all or part of the sequence, or sequence-conservative or function-conservative variants thereof, may be employed in practicing the present invention.

In a similar manner, additional sequences derived from the 5' and 3' flanking regions of sequence encoding the protein, including regulatory sequences, may be isolated, and the nucleotide sequence determined.

### Polypeptides

Both the naturally occurring and recombinant forms of the polypeptides described herein, including both glycosylated and non-glycosylated forms are encompassed by the invention. The polypeptides of the present invention, including function-conservative variants, may be isolated from human monocytes, or from heterologous organisms or cells (e.g., bacteria, fungi, insect, plant, and mammalian cells) into which a protein-coding sequence has been introduced and expressed. The proteins described herein, or portions thereof, also may be expressed as fusions with other proteins. The polypeptides may be chemically synthesized by commercially available automated procedures, including, without limitation, exclusive solid phase synthesis, partial solid phase methods, fragment condensation or classical solution synthesis. The polypeptides can also, advantageously, be made by *in vitro* translation.

Methods for polypeptide purification are well known in the art, including, without limitation, preparative disc-gel electrophoresis, isoelectric focusing, sucrose density gradient centrifugation, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against a protein or against peptides derived therefrom can be used as purification reagents. Other purification methods are possible.

The present invention also encompasses derivatives and homologues of the polypeptides specifically disclosed herein. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The polypeptides of the invention find use, e.g., for binding studies, for construction and expression of modified molecules, for structure/function studies and for the preparation of polyclonal and monoclonal antibodies. Polypeptides useful as immunogenic components for preparing antibodies or as targets for binding agent studies are at least five or more residues in length. Preferably, the polypeptides comprise at least about 12, more preferably at least about 20, and most preferably at least about 30 or more residues. Methods for obtaining these polypeptides are well known and are explained in *Immunochemical Methods in Cell and Molecular Biology,* 1987 (Mayer and Waler, eds; Academic Press, London); Scopes, 1987, *Protein Purification: Principles and Practice,* Second Edition (Springer-Verlag, N.Y.) and *Handbook of Experimental Immunology,* 1986, Volumes I-IV (Weir and Blackwell, eds.).

Having isolated one member of a binding partner of a specific interaction, methods exist for isolating the counter-partner. See, e.g., Gearing *et al.,* 1989, *EMBO J.* **8**:3667-3676. Many methods of screening for binding activity are known by those skilled in the art and may be used to practice the invention. For example, an expression library can be screened for specific binding to the protein, e.g., by cell sorting, or other screening to detect subpopulations which express such a binding component. See, e.g., Ho *et al.,* 1993, *Proc. Natl. Acad. Sci. USA* **90**:11267-11271. Alternatively, a panning method may be used. See, e.g., Seed and Aruffo, 1987, *Proc. Natl. Acad. Sci. USA* **84**:3365-3369. A two-hybrid selection system may also be applied making appropriate constructs with the available protein sequences. See, e.g., Fields and Song, 1989, *Nature* **340**:245-246. Several methods of automated assays have been developed in recent years so as to permit screening of tens of thousands of compounds in a short period of time.

### Physical Variants

This invention also encompasses proteins or peptides having substantial amino acid sequence similarity with an amino acid sequence of a SEQ ID NOS: 2, 4 or 6. Variants exhibiting substitutions, e.g., 20 or fewer, preferably 10 or fewer, and more preferably 5 or fewer substitutions, are encompassed. Where the substitutions are conservative substitutions, the variants will share immunogenic or antigenic similarity or cross-reactivity with a corresponding natural sequence protein. Natural variants include individual, allelic, polymorphic, strain, or species variants.

Amino acid sequence similarity, or sequence identity, is determined by optimizing residue matches, if necessary, by introducing gaps as required. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Homologous amino acid sequences include natural allelic and interspecies variations in each respective protein sequence. Typical homologous proteins or peptides will have from 50-100% similarity (if gaps can be introduced), to 75-100% similarity (if conservative substitutions are included) with the amino acid sequence of the relevant protein. Identity measures will be at least about 50%, generally at least 60%, more generally at least 65%, usually at least 70%, more usually at least 75%, preferably at least 80%, and more preferably at least 80%, and in particularly preferred embodiments, at least 85% or more. See also Needleham *et al.,* 1970, *J. Mol. Biol.* 48:443-453; Sankoff *et al.,* 1983, *Time Warps, String Edits, and Macromolecules: The Theory and Practice of Sequence Comparison* Chapter One, Addison-Wesley, Reading, MA; and software packages from IntelliGenetics, Mountain View, CA; and the University of Wisconsin Genetics Computer Group (GCG), Madison, WI.

Nucleic acids encoding the corresponding proteins will typically hybridize to SEQ ID NOS: 1, 3 or 5 under stringent conditions. For example, nucleic acids encoding the respective proteins will typically hybridize to the nucleic acid of SEQ ID NOS: 1, 3 or 5 under stringent hybridization conditions, while providing few false positive hybridization signals. Generally, stringent conditions are selected to be about 10° C lower than the thermal melting point (Tm) for the sequence being hybridized to at a defined ionic strength and pH. The Tm is the temperature (under defined ionic strength and pH) at which 50% of the target sequence hybridizes to a perfectly matched probe. Typically, stringent conditions will be those in which the salt concentration in wash is about 0.02 molar at pH 7 and the temperature is at least about 50° C. Other factors may significantly affect the stringency of hybridization, including, among others, base composition and size of the complementary strands, the presence of organic solvents such as formamide, and the extent of base mismatching. A preferred embodiment will include nucleic acids that will bind to disclosed sequences in 50% formamide and 20-50 mM NaCl at 42°C.

An isolated nucleic acid can be readily modified by nucleotide substitutions, nucleotide deletions, nucleotide insertions, and inversions of nucleotide stretches. These modifications result in novel DNA sequences which encode these antigens, their derivatives, or proteins having highly similar physiological, immunogenic, or antigenic activity.

Modified sequences can be used to produce mutant antigens or to enhance expression. Enhanced expression may involve gene amplification, increased transcription, increased translation, and other mechanisms. Such mutant protein derivatives include predetermined or site-specific mutations of the respective protein or its fragments. "Mutant protein" encompasses a polypeptide otherwise falling within the homology definition of the proteins as set forth above, but having an amino acid sequence which differs from that of the protein as found in nature, whether by way of deletion, substitution, or insertion. In particular, "site specific mutant protein" generally includes proteins having significant similarity with a protein having a sequence of SEQ ID NOS: 2, 4 or 6. Generally, the variant will share many physicochemical and biological activities, e.g., antigenic or immunogenic, with those sequences, and in preferred embodiments, contain most or all of the disclosed sequence.

Glycosylation alterations are included, e.g., made by modifying the glycosylation patterns of a polypeptide during its synthesis and processing, or in further processing steps. Particularly preferred means for accomplishing this are by exposing the polypeptide to glycosylating enzymes derived from cells that normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine, or other moieties, including ribosyl groups or cross-linking reagents. Also, proteins comprising substitutions are encompassed, which should retain substantial immunogenicity, to produce antibodies that recognize a protein of SEQ ID NOS: 2, 4 or 6. Typically, these proteins will contain less than 20 residue substitutions from the disclosed sequence, more typically less than 10 substitutions, preferably less than 5, and more preferably less than three. Alternatively, proteins that begin and end at structural domains will usually retain antigenicity and cross immunogenicity.

A major group of derivatives are covalent conjugates of the proteins described herein or fragments thereof with other proteins or polypeptides. These derivatives can be synthesized in recombinant culture such as N- or C-terminal fusions or by the use of agents known in the art for their usefulness in cross-linking proteins through reactive side groups. Preferred protein derivatization sites with cross-linking agents are at free amino groups, carbohydrate moieties, and cysteine residues.

Fusion polypeptides between these proteins and other homologous or heterologous proteins are also provided. Heterologous polypeptides may be fusions between different surface markers, resulting in, e.g., a hybrid protein. Likewise, heterologous fusions may be constructed which would exhibit a combination of properties or activities of the derivative proteins. Typical examples are fusions of a reporter polypeptide, e.g., luciferase, with a segment or domain of a protein, e.g., a receptor-binding segment, so that the presence or location of the fused protein may be easily determined. See, e.g., U.S. Patent No. 4,859,609. Other gene fusion partners include bacterial β-galactosidase, trpE, Protein A, β-lactamase, alpha amylase, alcohol dehydrogenase, and yeast alpha mating factor. See, e.g., Godowski *et al.,* 1988, *Science* **241**:812-816.

Such polypeptides may also have amino acid residues that have been chemically modified by phosphorylation, sulfonation, biotinylation, or the addition or removal of other moieties, particularly those that have molecular shapes similar to phosphate groups. In some embodiments, the modifications will be useful labeling reagents, or serve as purification targets, e.g., affinity ligands.

This invention also contemplates the use of derivatives of these proteins other than variations in amino acid sequence or glycosylation. Such derivatives may involve covalent or aggregative association with chemical moieties. These derivatives generally fall into the three classes: (1) salts, (2) side chain and terminal residue covalent modifications, and (3) adsorption complexes, for example with cell membranes. Such covalent or aggregative derivatives are useful as immunogens, as reagents in immunoassays, or in purification methods such as for affinity purification of ligands or other binding ligands. For example, a protein antigen can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of antibodies. The proteins can also be labeled with a detectable group, e.g., radioiodinated by the chloramine T procedure, covalently bound to rare earth chelates, or conjugated to another fluorescent moiety for use in diagnostic assays. Purification of these proteins may be accomplished by immobilized antibodies.

### Antibodies

The immunogenic components of this invention, as described above, are useful as antigens for preparing antibodies by standard methods. Such immunogenic components can be produced by proteolytic cleavage of larger polypeptides or by chemical synthesis or recombinant technology and are thus not limited by proteolytic cleavage sites. Preferably, smaller immunogenic components will first be rendered more immunogenic by cross-linking or by coupling to an immunogenic carrier molecule (i.e., a macromolecule having the property of independently eliciting an immunological response in a host animal, to which the immunogenic components of the invention can be covalently linked). Cross-linking or conjugation to a carrier molecule may be required because small polypeptide fragments sometimes act as haptens (molecules which are capable of specifically binding to an antibody but incapable of eliciting antibody production, i.e., they are not immunogenic). Conjugation of such fragments to an immunogenic carrier molecule renders them immunogenic through what is commonly known as the "carrier effect".

Antibodies according to the present invention include polyclonal and monoclonal antibodies. The antibodies may be elicited in an animal host by immunization with immunogenic components of the invention or may be formed by *in vitro* immunization (sensitization) of immune cells. The immunogenic components used to elicit the production of antibodies may be isolated from human cells (e.g., human dendritic cells) or chemically synthesized. The antibodies may also be produced in recombinant systems programmed with appropriate antibody-encoding DNA. Alternatively, the antibodies may be constructed by biochemical reconstitution of purified heavy and light chains.

The antibodies of this invention can be purified by standard methods, including but not limited to, preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in *The Art of Antibody Purification,* 1989, Amicon Division, W.R. Grace & Co. General protein purification methods are described in *Protein Purification: Principles and Practice,* R.K. Scopes, Ed., 1987, Springer-Verlag, New York, NY.

Suitable adjuvants for the vaccination of animals include but are not limited to Adjuvant 65 (containing peanut oil, mannide monooleate and aluminum monostearate); Freund's complete or incomplete adjuvant; mineral gels such as aluminum hydroxide, aluminum phosphate and alum; surfactants such as hexadecylamine, octadecylamine, lysolecithin, dimethyldioctadecyl-ammonium bromide, N,N-dioctadecyl-N',N'-bis(2-hydroxymethyl) propane-diamine, methoxyhexadecylglycerol and pluronic polyols; polyanions such as pyran, dextran sulfate, poly IC, polyacrylic acid and carbopol; peptides such as muramyl dipeptide, dimethylglycine and tuftsin; and oil emulsions. The immunogenic components could also be administered following incorporation into liposomes or other microcarriers. Information concerning adjuvants and various aspects of immunoassays are disclosed, e.g., in the series by P. Tijssen, 1987, *Practice and Theory of Enzyme Immunoassays,* 3rd Edition, Elsevier, New York.

Serum produced from animals thus immunized can be used directly. Alternatively, the IgG fraction can be separated from the serum using standard methods such as plasmaphoresis or adsorption chromatography using IgG specific adsorbents such as immobilized Protein A.

Hybridomas of the invention used to make monoclonal antibodies against the immunogenic components of the invention are produced by well-known techniques. Usually, the process involves the fusion of an immortalizing cell with a B-lymphocyte that produces the desired antibody. Alternatively, non-fusion techniques for generating immortal antibody-producing cell are possible, and come within the purview of the present invention, e.g., virally-induced transformation, Casali *et al.,* 1986, *Science* **234**:476. Immortalizing cell are usually transformed mammalian cells, particularly myeloma cells of rodent, bovine, and human origin. Most frequently, rat or mouse myeloma cell lines are employed as a matter of convenience and availability.

Techniques for obtaining the appropriate lymphocytes from mammals injected with the immunogenic components are well known. Generally, peripheral blood lymphocytes (PBLs) are used if cells of human origin are desired, or spleen cells or lymph node cells are used if non-human mammalian sources are desired. A host animal is injected with repeated dosages of a preferably purified immunogenic component, and the animal is permitted to generate the desired antibody-producing cells before these are harvested for fusion with the immortalizing cell line. Techniques for fusion are also well known in the art, and in general involve mixing the cells with a fusing agent, such as polyethylene glycol.

Hybridomas are selected by standard procedures, such as HAT (hypoxanthine-aminopterin-thymidine) selection. From among these hybridomas, those secreting the desired antibody are selected by assaying their culture medium by standard immunoassays, such as Western blotting, ELISA (enzyme-linked immunosorbent assay), RIA (radioimmunoassay), or the like. Antibodies are recovered from the medium using standard protein purification techniques, Tijssen, 1985, *Practice and Theory of Enzyme Immunoassays,* Elsevier, Amsterdam.

Many references are available for guidance in applying any of the above techniques: Kohler *et al.,* 1980, *Hybridoma Techniques,* Cold Spring Harbor Laboratory, New York; Tijssen, 1985, *Practice and Theory of Enzyme Immunoassays,* Elsevier, Amsterdam; Campbell, 1984, *Monoclonal Antibody Technology,* Elsevier, Amsterdam; Hurrell, 1982, *Monoclonal Hybridoma Antibodies: Techniques and Applications,* CRC Press, Boca Raton, FL. Monoclonal antibodies can also be produced using well-known phage library systems.

The use and generation of antibody fragments is also well known, e.g., Fab fragments: Tijssen, 1985, *Practice and Theory of Enzyme Immunoassays,* Elsevier, Amsterdam; Fv fragments: Hochman *et al.,* 1973, *Biochemistry* **12**:1130; Sharon *et al.,* 1976, *Biochemistry* **15**:1591; Ehrlich *et al.,* U.S. Patent No. 4,355,023; and antibody half molecules: Auditore-Hargreaves, U.S. Patent No. 4,470,925. These also may be useful in immunoassays.

These antibodies, whether polyclonal or monoclonal, can be used, e.g., in an immobilized form bound to a solid support by well known methods, to isolate and purify the immunogenic components by immunoaffinity chromatography. The antibodies are useful as probes to distinguish tissue and cell type distribution. The antibodies may be used to screen expression libraries for particular expression products. Usually the antibodies used in such a procedure will be labeled with a moiety allowing easy detection of presence of antigen by antibody binding. Antibodies to proteins may be used for the analysis or, or identification of specific cell population components which express the respective protein. By assaying the expression products of cells expressing the proteins described herein it is possible to diagnose disease, e.g., immune-compromised conditions, monocyte-depleted conditions, or overproduction of monocytes. Antibodies raised against the proteins will also be useful to raise anti-idiotypic antibodies. These will be useful in detecting or diagnosing various immunological conditions related to expression of the respective antigens. The present invention encompasses antibodies that specifically recognize monocyte-derived immunogenic components. Such antibodies can be used conventionally, e.g., as reagents for purification of monocyte cell components, or in diagnostic applications.

### Diagnostic Applications

The invention encompasses compositions, methods, and kits useful in clinical settings for the qualitative or quantitative diagnosis, i.e., detection of specific components in a biological sample. These applications utilize nucleic acids, peptides/polypeptides, or antibodies specific for the components described herein. Both antibody-based and nucleic acid-based diagnostic methods, including PCR-based diagnostic methods are contemplated. Detection of the level of certain types of dendritic cells present in a sample could be important for diagnosis of certain aberrant disease conditions. For instance, in breast carcinoma tissues, markers specific for maturation stages of dendritic cells showed that dendritic cells present within the tumor were frozen in an immature stage thus illustrating a possible mechanism for tumor escape Bell D., *et al.* "In breast carcinoma tissue, immature dendritic cells reside within the tumor, whereas mature dendritic cells are located in peritumoral areas." *J Exp Med.* 1999 Nov 15, **190**(10):1417-26).

Both the naturally occurring and the recombinant form of the proteins of this invention are particularly useful in kits and assay methods which are capable of screening compounds for binding activity to the proteins.

In nucleic-acid-type diagnostic methods, the sample to be analyzed may be contacted directed with the nucleic acid probes. Probes include oligonucleotides at least 12 nucleotides, preferably at least 18, and most preferably 20-35 or more nucleotides in length. Alternatively, the sample may be treated to extract the nucleic acids contained therein. It will be understood that the particular method used to extract DNA will depend on the nature of the biological sample. The resulting nucleic acid from the sample may be subjected to gel electrophoresis or other size separation techniques, or, the nucleic acid sample may be immobilized on an appropriate solid matrix without size separation or used for PCR.

Kits suitable for antibody-based diagnostic applications typically include one or more of the following components:
(i) Antibodies: The antibodies may be pre-labeled; alternatively, the antibody may be unlabelled and the ingredients for labeling may be included in the kit in separate containers, or a secondary, labeled antibody is provided; and
(ii) Reaction components: The kit may also contain other suitably packaged reagents and materials needed for the particular immunoassay protocol, including solid-phase matrices, if applicable, and standards.

Kits suitable for nucleic acid-based diagnostic applications typically include the following components:
(i) *Probe DNA*: The probe DNA may be pre-labeled; alternatively, the probe DNA may be unlabelled and the ingredients for labeling may be included in the kit in separate containers; and
(ii) *Hybridization reagents:* The kit may also contain other suitably packaged reagents and materials needed for the particular hybridization protocol, including solid-phase matrices, if applicable, and standards.

PCR based diagnostic kits are also contemplated and are encompassed by the invention.

The kits referred to above may include instructions for conducting the test. Furthermore, in preferred embodiments, the diagnostic kits are adaptable to high-throughput and/or automated operation.

### Therapeutic Applications

The invention also provides reagents that may exhibit significant therapeutic value. The proteins (naturally occurring or recombinant), fragments thereof, and antibodies thereto, along with compounds identified as having binding affinity to the proteins, may be useful in the treatment of conditions associated with abnormal physiology or development. For example, a disease or disorder associated with abnormal expression or abnormal signaling by a dendritic cell, e.g., as an antigen-presenting cell, is a target for an agonist or antagonist of the protein. The proteins likely play a role in regulation or development of hematopoietic cells, e.g., lymphoid cells, which affect immunological responses, e.g., antigen presentation and the resulting effector functions.

Recombinant dendritic cell-derived proteins or antibodies of the invention may be purified and then administered to a patient. These reagents can be combined for therapeutic use with additional active or inert ingredients, e.g., in conventional pharmaceutically acceptable carriers or diluents, e.g., immunogenic adjuvants, along with physiologically innocuous stabilizers and excipients. In particular, these may be useful in a vaccine context, where the antigen is combined with one of these therapeutic versions of agonists or antagonists. These combinations can be sterile filtered and placed into dosage forms as by lyophilization in dosage vials or storage in stabilized aqueous preparations. This invention also contemplates use of antibodies or binding fragments thereof, including forms which are not complement binding.

Drug screening using antibodies or receptor or fragments thereof can identify compounds having binding affinity to these dendritic cell-derived proteins, including isolation of associated components. Subsequent biological assays can then be utilized to determine if the compound blocks or antagonizes the activity of the protein. Likewise, a compound having intrinsic stimulating activity might activate the cell through the protein and is thus an agonist. This invention further contemplates the therapeutic use of antibodies to the proteins as antagonists.

The quantities of reagents necessary for effective therapy will depend upon many different factors, including means of administration, target site, physiological state of the patient, and other medicants administered. Thus, treatment dosages should be titrated to optimize safety and efficacy. Typically, dosages used *in vitro* may provide useful guidance in the amounts useful for in situ administration of these reagents. Animal testing of effective doses for treatment of particular disorders will provide further predictive indication of human dosage. Various considerations are described, e.g., in Gilman, et al. (eds.) (1990) *Goodman and Gilman's: The Pharmacological Bases of Therapeutics* (8th ed.) Pergamon Press; and (1990) *Remington's Pharmaceutical Sciences* (17th ed.) Mack Publishing Co., Easton, PA. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the *Merck Index,* Merck & Co., Rahway, NJ. Dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or a slow release apparatus will often be utilized for continuous administration.

The proteins, antagonists, and agonists could be administered directly to the host to be treated or, depending on the size of the compounds, it may be desirable to conjugate them to carrier proteins such as ovalbumin or serum albumin prior to their administration. Therapeutic formulations may be administered in many conventional dosage formulations. While it is possible for the active ingredient to be administered alone, it is preferable to present it as a pharmaceutical formulation. Formulations typically comprise at least one active ingredient, as defined above, together with one or more acceptable carriers thereof. Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds.) (1990) *Goodman and Gilman's: The Pharmacological Bases of Therapeutics* (8th ed.) Pergamon Press; and (1990) *Remington's Pharmaceutical Sciences* (17th ed.) Mack Publishing Co., Easton, PA; Avis, et al. (eds.) (1993) *Pharmaceutical Dosage Forms: Parenteral Medications* Dekker, NY; Lieberman, et al. (eds.) (1990) *Pharmaceutical Dosgae Forms: Tablets* Dekker, NY; and Lieberman, et al. (eds.) (1990) *Pharmaceutical Dosage Forms: Disperse Systems* Dekker, NY. The therapy of this invention may be combined with or used in association with other chemotherapeutic or chemopreventive agents.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. An isolated polypeptide comprising an amino acid sequence derived from SEQ ID NOS: 2,4 or 6.

2. The polypeptide of claim 1 comprising the amino acid sequence of the mature protein.

3. An isolated nucleic acid comprising a nueclotide sequence encoding an amino acid sequence derived from SEQ ID NO: 2, 4 or 6.

4. The nucleic acid of claim 3 wherein the nucloetide sequence encodes the mature protein.

5. The nucleic acid of claim 4 comprising the nucleotide sequence set forth in SEQ ID NO: 1, 3 or 5.

6. A fusion protein comprising the polypeptide of claim 1.

7. A binding compound which specifically binds to the polypeptide of claim 1.

8. The binding compound of claim 7 wherein said binding compound is an antibody or antibody fragment.

9. The binding compound of claim 8 wherein said antibody is a monoclonal antibody.

10. An expression vector comprising the nucleic acid of claim 3.

11. An expression vector comprising the nucleic acid of claim 5.

12. A host cell comprising the vector of claim 10.

13. A process for recombinantly producing a polypeptide comprising culturing the host cell of claim 12 under conditions in which said polypeptide is expressed.

14. A method for detecting a specific nucleic acid sequence in a sample, said method comprising the steps of:
a) contacting a sample suspected to contain a specific nucleic acid sequence with a probe comprising a nucleic acid sequence comprising at least 8 consecutive nucleotides selected from SEQ ID NO: 1, 3 or 5 under conditions in which a hybrid can form between said probe and the specific nucleic acid in said sample;
b) detecting any hybrid formed in step (a),wherein detecting of said hybrid indicates the presence of the specific nucleic acid sequence in said sample.

15. The method of claim 14 further comprising amplifying said specific sequence in said sample prior to said detecting step.

16. A method for detecting a specific antigenic component in a sample, said method comprising the steps of:
a) contacting a sample suspected to contain a specific antigenic component encoded by an amino acid sequence derived from SEQ ID NO: 2, 4 or 6 with an antibody or antibody fragment specific for said component under conditions in which a stable antigen-antibody complex can form between said antibody or said antibody fragment and said antigenic component in said sample; and
b) detecting any antigen-antibody complex formed in step (a), wherein detection of an antigen-antibody complex indicates the presence of said antigenic component in said sample.

17. A method of screening for candidate therapeutic agents comprising:
a) selecting as a target sequence a polypeptide having an amino acid sequence derived from SEQ ID NO: 2, 4 or 6.
b) contacting a test compound with said target sequence; and
c) selecting as said candidate therapeutic agent those test compounds which bind to the target sequence.
